# EUROPEAN PATENT APPLICATION

(11) **EP 4 657 378 A1**
(43) Date of publication of application: **03.12.2025**
(21) Application number: 24178872.8
(22) Date of filing: 29.05.2024
(51) Int. Cl.: G06T 11/00

(54) **TIME-RESOLVED MEDICAL IMAGING**

(71) Applicant: Siemens Healthineers AG, 91301 Forchheim (DE)
(72) Inventor: Dickmann, Jannis, 91052 Erlangen (DE); Vega, Fernando, 91056 Erlangen (DE); Wohlfahrt, Patrick, 91054 Erlangen (DE)
(74) Representative: Siemens Healthineers Patent Attorneys

(57) **Abstract**

For generating a reconstructed 4D-volume in time-resolved medical imaging, a plurality of temporally ordered imaging datasets (5a, 5b) representing an imaged object corresponding to a motion of the object is received. Each imaging dataset of the plurality of imaging datasets (5a, 5b) is assigned to a first group (5a) or to a second group (5b) depending on a noise-affecting imaging parameter used for generating the respective imaging dataset. Each imaging dataset of the first group (5a) is denoised using a denoising algorithm. A 4D-volume of the object is generated based on each denoised imaging dataset of the first group (5a) and based on each imaging dataset of the second group (5b).

## Description

The present invention is directed to a computer-implemented method for four-dimensional, 4D-, reconstruction in time-resolved medical imaging, wherein a plurality of temporally ordered imaging datasets representing an imaged object and corresponding to a motion of the object is received. The invention is further directed to a corresponding method for time-resolved medical imaging comprising and to a data processing system adapted to carry out said computer-implemented method. The invention is further directed to a system for time-resolved medical imaging comprising said data processing system and to corresponding computer program products.

Time-resolved medical imaging, for example 4D computed tomography imaging, 4DCT, or 4D magnetic resonance imaging, 4DMRI, may be used for various applications including for example for planning radiotherapy treatment of moving tumors, for example in the lung or liver. Therein, 3D image data are acquired at various time points during a motion, for example a cyclic motion, for example during the patient's respiratory cycle. The image data may for example be used to contour the spatial extent of the tumor as it moves over time.

In this context, and generally in time-resolved medical imaging, a high image quality, for example in terms of a high signal-to-noise-ratio, SNR, is beneficial, in particular in low-contrast regions as the liver. The SNR may for example be increased by adjusting respective imaging parameters, for example increasing the X-ray dose in 4DCT or prolonging the acquisition time in 4DMRI. However, an increased X-ray dose or a longer acquisition time are also undesirable.

Denoising approaches are described in literature and applied to 4DCT data, for example in the publication A. Inouse et al.: "Diagnostic Performance in Low- and High-Contrast Tasks of an Image-Based Denoising Algorithm Applied to Radiation Dose-Reduced Multiphase Abdominal CT Examinations", AJR, vol 220, 1 (2022). However, they may negatively impact spatial resolution and/or geometrical accuracy due to limited performance of internal algorithms such as deep-learning approaches or non-rigid image registration.

Document DE 10 2016 202 605 A1 describes a method for respiration-correlated computed tomographic image acquisition, wherein a patient-specific respiratory curve is recorded and evaluated online, and wherein a computed tomographic scan is controlled synchronously with the patient-specific respiratory curve depending on the results of the online evaluation.

It is an objective of the present invention to increase the image quality in time-resolved medical imaging, which overcomes said drawbacks of existing approaches at least partially.

This objective is achieved by the subject matter of the independent claim. Further implementations and preferred embodiments are subject matter of the dependent claims, the description and the figures.

The invention is based on the idea to assign each imaging dataset of a plurality of temporally ordered imaging datasets to a first group or to a second group depending on a noise affecting imaging parameter used for generating the respective imaging dataset. The imaging datasets of the first group are denoised but a reconstructed 4D-volume is generated based on the denoised imaging datasets as well as the imaging datasets of the second group.

According to an aspect of the invention, a computer-implemented method for generating a reconstructed four-dimensional, 4D-, volume in time-resolved medical imaging is provided. Therein, a plurality of temporally ordered imaging datasets representing an imaged object, is received. The plurality datasets corresponds to, in particular has been generated during, a motion of the object. Each imaging dataset of the plurality of imaging datasets is assigned to a first group or to a second group, in particular either to the first group or the second group, depending on a noise affecting imaging parameter used for generating the respective imaging dataset. Each imaging dataset of the first group is denoised using a denoising algorithm. A reconstructed 4D-volume of the object is generated based on the denoised imaging datasets of the first group and based on the imaging datasets of the second group.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for generating a reconstructed 4D-volume in time-resolved medical imaging, which is not purely computer-implemented, is obtained by including respective steps of generating the plurality of temporally ordered imaging datasets by a respective imaging apparatus.

The reconstructed 4D-volume can be understood as a time-resolved or time-dependent 3D-reconstruction. In other words, the 4D-volume comprises a respective 3D-reconstruction for a plurality of time steps or time intervals, respectively, which correspond to the respective data acquisition time intervals during which the imaging datasets have been generated. For example, the 4D-volume comprises a respective 3D-reconstruction for each of the plurality of temporally ordered imaging datasets, irrespective of whether the respective image dataset is assigned to the first group or the second group. It is also possible that the 4D-volume comprises a respective 3D-reconstruction only for a subset of the plurality of temporally ordered imaging datasets. Therein, however, said subset comprises in general imaging datasets assigned to the first group as well as imaging datasets assigned to the second group.

The generation of the 3D-reconstructions and/or the reconstructed 4D-volume per se may be carried out using known methods for medical image reconstruction, for example in CT, cone-beam CT, CBCT, or MRI, depending on the actual use case. However, according to the invention, the denoised imaging datasets of the first group are used as a basis for the generating 4D-volume. The imaging datasets of the second group may, for example, not be denoised. In other words, the 4D-volume may be generated based on each denoised imaging dataset of the first group and based on each non-denoised imaging dataset of the second group. This, in combination with the grouping according to the noise-affecting imaging parameter used for generating the respective imaging datasets, allows to achieve an increased image quality of the 4D-volume. For example, a reconstructed 4D-volume may comprise or consist of respective 3D-reconstructions.

For example, the denoising may be realized as a non-rigid registration to a target volume and then averaging with the target volume. The imaging datasets of the first group and of the second group may for example be used to denoise the imaging datasets first group. The second group may not necessarily be denoised.

For example, in X-ray based imaging such as 4DCT or 4D-CBCT, the noise-affecting imaging parameter may affect an X-ray dose, such as a tube current of an X-ray tube, wherein the second group corresponds to imaging datasets generated using a higher tube current than for generating the imaging datasets of the first group. The higher tube current for the imaging datasets of the second group increases the SNR for these imaging datasets, while at the same time, the lower tube current for the imaging datasets of the first group limits an increase in the X-ray dose. Analogously, in MR imaging, the noise-affecting imaging parameter may be an acquisition time or a parameter affecting the acquisition or reconstruction time used for generating the respective imaging dataset. Also here, an increased acquisition time leads to an increased SNR on the one hand but to an increased overall time for the procedure resulting in potential motion-induced image artefacts. Similarly, sub-sampling or deep learning methods during the reconstruction may decrease reconstruction time, but increase SNR or other artifacts. Thus, here the second group corresponds for example to imaging datasets generated using a higher acquisition or reconstruction time than for generating the imaging datasets of the first group. This may be extended analogously to other noise-affecting imaging parameters and/or other medical imaging techniques or modalities. The noise-affecting imaging parameter used for generating the respective imaging dataset is, for example, received for each imaging datasets of the plurality of imaging datasets.

As mentioned above, the imaging datasets of the second group may not be denoised at all when using them for generating the 4D-volume. This does not exclude, however, that imaging datasets of the second group are used as auxiliary data for denoising the imaging datasets of the first group.

It is, however, also possible that the imaging datasets of the second group are denoised using a further denoising algorithm, which differs from the denoising algorithm used for denoising the imaging datasets of the first group. The denoising algorithm and the further denoising algorithm may differ methodologically or may only differ in one or more parameters. In particular, the denoising algorithm and the further denoising algorithm may differ in their denoising strength, such that the denoising algorithm has a greater denoising strength than the further denoising algorithm. Depending on the concrete implementation, the denoising strength may be affected by different parameters.

Each imaging dataset of the plurality of imaging datasets may comprise or consist of a respective 3D-reconstruction. It is also possible that an imaging dataset of the plurality of imaging datasets comprises raw data or pre-processed raw data that is both suitable and sufficient to be used to generate a respective 3D-reconstruction. In case of MRI use cases, the imaging datasets may be given in k-space or in image space or in a hybrid space.

In particular, a given imaging dataset of the plurality of imaging datasets may comprise or may be generated based on one or more subsets. In X-ray imaging, in particular CT, said subsets may correspond to different angulation states or imaging directions, while in MRI, the subsets may for example correspond to different portions of the k-space, et cetera.

The motion may for example be a cyclic motion. The cyclic motion can be caused by various phenomena including, for example, a respiratory motion or a cardiac motion, in case the imaged object is a human or animal. In other words, in some embodiments, the object is a patient, and the cyclic motion corresponds to a respiratory motion of the patient or to a cardiac motion of the patient.

According to at least one embodiment, the denoising algorithm is applied to input data, which contains at least one of the imaging datasets of the first group and at least one of the imaging datasets of the second group.

For example, the input data may contain all imaging datasets of the first group and all imaging datasets of the second group. This may be particularly beneficial in case the denoising algorithm is based on a trained machine learning model, MLM, for example a trained artificial neural network, ANN. In particular, the denoising algorithm may have to be applied only once to generate all denoised imaging datasets of the first group. It is also possible that the denoising algorithm denoises all imaging datasets of the input data, for example also all imaging datasets of the second group. In this case, the denoised imaging datasets of the second group may for example be discarded and not be used for generating the 4D reconstruction. In this way, it is avoided that denoising artifacts are introduced although the denoising has not been necessary in the first place due to the higher image quality of the imaging datasets of the second group. Furthermore, the information contained in the high-quality imaging datasets of the second group is exploited for the denoising of the imaging datasets of the first group and also for generating the 4D-volume in the next step.

It is, however, also possible that the denoising algorithm is applied individually for each imaging dataset of the first group. Also in this case, the remaining imaging datasets of the plurality of imaging datasets, that is all imaging datasets of the first group and the second group except for the imaging dataset currently being denoised, may be used in the denoising, for example as auxiliary data, in particular in denoising algorithms that are based on weighted averaging of multiple imaging datasets.

In other words, in such embodiments, at least one of the imaging datasets of the second group is used for denoising at least one of the imaging datasets of the first group. The denoised imaging dataset of the first group is used for generating 4D-volume, while, for example, the non-denoised imaging dataset of the second group is used for generating the 4D-volume. For example, the 4D-volume may comprise the non-denoised imaging datasets of the second group and the denoised imaging datasets of the first group.

According to several embodiments, the denoising algorithm comprises a trained MLM for denoising in medical imaging.

Such MLMs are known in the context of various medical imaging techniques such as X-ray based projection imaging, CT and MRI. As mentioned above, it is possible, that the output of the MLM comprises denoised versions of all input image datasets. However, in this case, only the denoised imaging datasets of the first group but not the denoised imaging datasets of the second group are for example used for generating the 4D-volume.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network, GAN.

According to several embodiments, for each imaging dataset of the first group, the following steps are carried out for denoising the respective imaging dataset of the first group: All remaining imaging datasets of the plurality of imaging datasets, that is all imaging datasets of the first group and the second group except for the respective imaging dataset of the first group currently being denoised, are registered to the respective imaging dataset of the first group currently being denoised. Denoising the respective imaging dataset of the first group comprises computing a weighted average of the respective imaging dataset of the first group and the registered imaging datasets, in particular all of the registered imaging datasets.

Such denoising algorithms are also known per se and can be used in the context of respective embodiments. However, according to the invention, such denoising algorithm is only used in order to denoise the imaging datasets of the first group but not those of the second group, which saves computational time and memory.

According to several embodiments, the plurality imaging datasets correspond to X-ray images or to computed tomography, CT-, datasets, for example 3D-CT-reconstructions, and the noise-affecting imaging parameter concerns an X-ray dose used for generating the respective imaging dataset.

The noise affecting imaging parameter may for example be given by or depend on a tube current of an X-ray tube of an X-ray imaging apparatus used for generating the respective imaging dataset. This includes conventional X-ray imaging apparatuses as well as C-arm devices and CT-apparatuses.

In this way, both the SNR and the applied X-ray dose may be tuned accurately by tuning the tube current during the data acquisition.

According to several embodiments, the plurality imaging datasets correspond to MRI-datasets and the noise-affecting imaging parameter concerns an acquisition or reconstruction time used for generating the respective imaging dataset.

The noise-affecting imaging parameter may for example be given by or depend on an acceleration factor of the acquisition scheme used for acquiring the respective MRI-dataset or a parameter specifying an acquisition method and/or k-space sampling scheme used for acquiring the respective MRI-dataset.

According to a further aspect of the invention, a method for time-resolved medical imaging is provided. Therein, a plurality of temporally ordered imaging datasets representing an imaged object is generated, in particular by an imaging apparatus, during s motion of the object. A computer-implemented method for generating a reconstructed 4D-volume in time-resolved medical imaging according to the invention is carried out based on said plurality of temporally ordered imaging datasets.

According to several embodiments, a motion state signal is generated indicating a current motion state of the motion, for example cyclic motion, and the imaging parameter is modulated depending on the motion state signal during the generation of the plurality of imaging datasets. The imaging datasets of the plurality of imaging datasets are assigned to the first group or to the second group depending on a respective value of the motion state signal.

The motion state signal may for example be generated based on a predicted respiratory curve for the patient in case the motion is a respiratory motion of the patient. Analogously, the motion state signal may in some implementations be generated based on a predicted coronary curve for the patient in case the motion is a cardiac motion of the patient.

The respective value of the motion state signal may for example be a mean value of the motion state signal during the respective data acquisition period for generating the respective imaging dataset. In case the motion state signal is a binary signal, it is also possible that the respective value of the motion state signal is the respective binary value, which is given for a longer time during the respective data acquisition period than the other binary value. It may also be ensured by means of controlling the generation of the motion state signal that a unique value of the motion state signal is defined for each imaging dataset.

Since the motion state signal is used for both modulating the noise-affecting imaging parameter and grouping the imaging datasets into the first group and the second group, respectively, a particularly reliable automatic correlation between the imaging datasets and noise-affecting imaging parameter is achieved in some cases.

For example, the motion state signal is generated as a binary signal assuming either a first value, for example 0, or a second value, for example 1. The motion state signal may be generated to assume the second value during a maximum inhale phase of the respiratory motion and/or during a maximum exhale phase of the respiratory motion. If the respective value is equal to the second value, then the respective imaging dataset is assigned to the second group and, for example, if the respective value is equal to the first value, then the respective imaging dataset is assigned to the first group.

The respiratory motion is a motion moving back and forth between a maximum inhale state and a maximum exhale state of the patient. A maximum inhale phase and the maximum exhale phase correspond to respective time periods during which the maximum inhale state and the maximum exhale state, respectively, are assumed. Since the motion of a part of the object of potential interest, such as tumor or the like, moves together with the respiratory motion, it may undergo a cyclic motion, whose maximum amplitudes are reached at the maximum inhale state and the maximum exhale state, respectively. Consequently, since the maximum amplitudes define the total spatial extent of the motion, the corresponding maximum inhale phase and maximum exhale phase may represent particularly relevant time periods for various use cases such as tumor contouring. Thus, it is particularly beneficial to capture the respective imaging datasets with a particularly high SNR, which is realized by modulating the noise-affecting imaging parameter according to the motion state signal as described.

For example, exactly one imaging dataset of the plurality of imaging datasets is generated during the maximum inhale phase and exactly one imaging dataset of the plurality of imaging datasets is generated during the maximum exhale phase. The motion state signal may for example be generated to assume the first value outside of the maximum inhale phase of the respiratory motion and outside of the maximum exhale phase of the respiratory motion. In this case, it follows that the second group consists of two imaging datasets, one corresponding to the maximum inhale phase and one corresponding to the maximum exhale phase.

The motion state signal may also be generated to assume the second value during a phase halfway between the maximum exhale phase and the maximum inhale phase, also denoted as halfway phase or mid-ventilation phase in the following. The halfway phase may represent a particularly relevant phase in some implementations and use cases as well. In particular, the position of the tumor during the halfway phase may be considered as an intermediate position during the motion. In some embodiments the motion state signal is generated to assume the second value during a maximum inhale phase of the respiratory motion and/or during a maximum exhale phase of the respiratory motion and/or during a phase halfway between the maximum exhale phase and the maximum inhale phase.

In some embodiments, the motion state signal is generated such that a resulting number of imaging dataset of the second group is less than a resulting number of imaging dataset of the first group.

For example, the number of imaging datasets of the first group may lie in the range from 5 to 16 and the number of imaging datasets of the second group may lie in the range from 1 to 4. For example, the number of imaging datasets of the plurality of imaging datasets may lie in the range from 6 to 20. An exemplary configuration may use 8 imaging datasets of the first group and 2 imaging datasets of the second group, distributed for example equally over a respiratory cycle.

According to several embodiments, the plurality imaging datasets is generated as X-ray images, in particular 2D projection images, or as CT-datasets, for example 3D-CT-reconstructions, by means of an X-ray imaging system and the imaging parameter is the tube current of the X-ray tube of the X-ray imaging system.

Further implementations of the method for time-resolved medical imaging according to the invention follow directly from the various embodiments of the computer-implemented method for generating a reconstructed 4D-volume in time-resolved medical imaging according to the invention and vice versa. In particular, individual features and corresponding explanations as well as advantages relating to the various implementations of the computer-implemented method according to the invention can be transferred analogously to corresponding implementations of the method according to the invention and vice versa.

According to a further aspect of the invention, a data processing system is provided. The data processing system is adapted to carry out a computer-implemented method for 4D-reconstruction in time-resolved medical imaging according to the invention.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a system for time-resolved medical imaging is provided. The system comprises a data processing system according to the invention and an imaging apparatus which is configured to generate the plurality of temporally ordered imaging datasets.

The imaging apparatus may for example be an X-ray imaging device, for example a C-arm apparatus or a CT-apparatus, or an MRI system.

Further implementations of the system for time-resolved medical imaging according to the invention follow directly from the various embodiments of the computer-implemented method and the method according to the invention and vice versa. In particular, individual features and corresponding explanations as well as advantages relating to the various implementations of the computer-implemented method or the method according to the invention can be transferred analogously to corresponding implementations of the system for time-resolved medical imaging according to the invention. In particular, the system for time-resolved medical imaging according to the invention is designed or programmed to carry out the method according to the invention. In particular, the system for time-resolved medical imaging according to the invention carries out the method according to the invention.

According to a further aspect of the invention, a computer program comprising instructions is provided. When the instructions are executed by a data processing system, the instructions cause the data processing system to carry out a computer-implemented method for 4D-volume in time-resolved medical imaging according to the invention.

The instructions may be provided as program code, for example. The program code can for example be provided as binary code or assembler and/or as source code of a programming language, for example C, and/or as program script, for example Python.

According to a further aspect of the invention, a further computer program comprising further instructions is provided. When the further instructions are executed by a system for time-resolved medical imaging according to the invention, in particular by the data processing system of the system for time-resolved medical imaging, the instructions cause the system for time-resolved medical imaging to carry out a method for time-resolved medical imaging according to the invention.

The further instructions may be provided as program code, for example. The program code can for example be provided as binary code or assembler and/or as source code of a programming language, for example C, and/or as program script, for example Python.

According to a further aspect of the invention, a computer-readable storage medium storing a computer program and/or a further computer program according to the invention is provided.

The computer program, the further computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary implementation of a system for time-resolved medical imaging according to the invention;
- FIG 2: shows as schematic representation of a plurality of temporally ordered imaging datasets; and
- FIG 3: shows schematically the motion of a part of an imaged object.

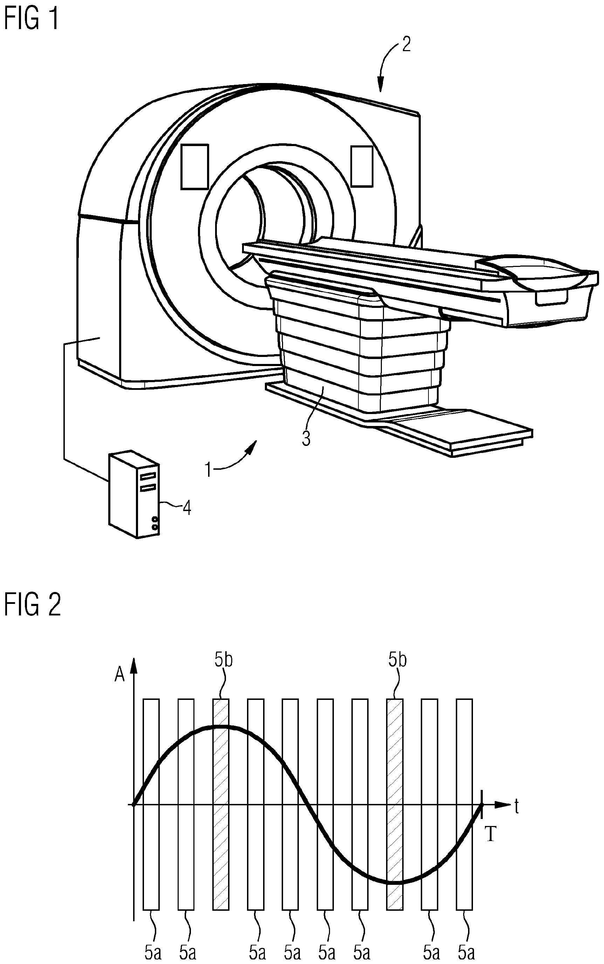

FIG 1 shows schematically an exemplary implementation of a system 1 for time-resolved medical imaging according to the invention. The system 1 comprises a data processing system 4 according to the invention and an imaging apparatus 2, which is configured to generate the plurality of temporally ordered imaging datasets 5a, 5b, as shown schematically in FIG 2, where t denotes the time and the rectangular blocks represent the imaging datasets 5a, 5b. The plurality of temporally ordered imaging datasets 5a, 5b represent an imaged object, for example a part of a patient, and correspond to a motion, for example a cyclic motion, of the object.

FIG 2 further shows an amplitude A of the motion of the imaged object, for example of a respiratory motion of the patient, over a period T. The extension along the axis of A of the rectangular blocks represent the imaging datasets 5a, 5b does not have a meaning here.

The data processing system 4 is adapted to carry out a computer-implemented method for 4D-volume in time-resolved medical imaging based on the plurality of temporally ordered imaging datasets 5a, 5b. Therein, the data processing system 4 assigns each imaging dataset of the plurality of imaging datasets 5a, 5b either to a first group 5a or to a second group 5b, depending on a noise-affecting imaging parameter used for generating the respective imaging dataset 5a, 5b. Each imaging dataset of the first group 5a is denoised using a denoising algorithm. A 4D-volume of the object is generated based on the denoised imaging datasets of the first group 5a and based on the imaging datasets of the second group 5b, in particular the imaging datasets of the second group 5b without denoising.

For example, the imaging apparatus 2 may be a CT scanner as shown as an example in FIG 1. The patient may be positioned on a patient table 3. The plurality of temporally ordered imaging datasets 5a, 5b may then for example be respective 3D-CT-reconstructions. The noise-affecting parameter may for example be a tube current of an X-ray tube of the CT scanner.

Consequently, the imaging datasets 5a of the first group may be generated using a first tube current and the imaging datasets 5b of the second group may be generated using a second tube current, which is greater than the first tube current. Thus, the SNR of the imaging datasets 5a of the first group is potentially less than the SNR of the imaging datasets 5b of the first group. Thus, generating the 4D-volume based on the denoised imaging datasets 5a of the first group and based on the imaging datasets 5b of the second group without denoising provides a very good trade-off between the totally applied X-ray dose and the image quality of the 4D-volume.

4DCT, in particular the 4D-volume obtained according to the invention, may for example be used for radiotherapy treatment planning of moving tumors, for example in the lung or liver. The 4D-volume is for example used to automatically contour the spatial extent of the tumor at each time point. For this, each 3D-reconstruction should have a sufficient image quality, in particular SNR, to allow for structures to be visible and easy to contour, in particular in low-contrast regions as the liver. This may result in a considerable imaging dose of 30-60 mGy for an entire respiratory 4DCT scan in conventional approaches, while only a limited SNR can be achieved in a single phase of the respiratory cycle.

In current clinical practice, a compromise between SNR and imaging dose is found for each tumor site, resulting in rather low-SNR images at rather high imaging dose, since imaging dose must be split into multiple time points. This can negatively impact the accuracy of contouring and hence treatment dose. Oftentimes, MRI or PET images are additionally obtained to aid contouring in low-contrast cases, which comes at an additional cost and is available only in few clinical sites. Furthermore, the multi-modality imaging approach for delineation of moving targets introduces additional challenges, since the images are potentially acquired at different times, for example on different days, in different patient immobilization, in non-corresponding motion states and/or with different contrast impressions that need to be aligned.

In several embodiments of the present invention, the overall 4DCT imaging dose may be lowered considerably, which also allows for a more frequent re-imaging during an adaptive radiotherapy workflow, for example.

In several embodiments, the invention allows to increase the SNR of the image data at each time point considerably and therefore leads to a better detectability of fine low-contrast lesions, for example.

Furthermore, in several embodiments, said advantages are achieved without a relevant reduction of spatial or temporal resolution and without hampering the geometric accuracy of CT scans, for example due to errors in deformable image registration, at least in the imaging datasets of the second group.

In several embodiments, a real-time respiratory-triggered modulation of the tube current during the CT acquisition is used in combination with a 4D denoising algorithm to achieve 4DCT scans at all time points with increased SNR and/or reduced imaging dose while ensuring full geometric accuracy at selected time points of the respiratory cycle.

The total number of the plurality of imaging datasets 5a, 5b is denoted by N, which is equal to 10 in the non-limiting example depicted in FIG 2. Each of the N imaging datasets 5a, 5b or the respective raw data is captured during a corresponding time interval. These time intervals may also be separated from each other as indicated in FIG 2. Prior to the data acquisition, M<N time intervals of particular importance may be selected. For example, M may be equal to two, wherein the two selected time intervals corresponding for example to a maximum inhale and the maximum exhale respiratory phase.

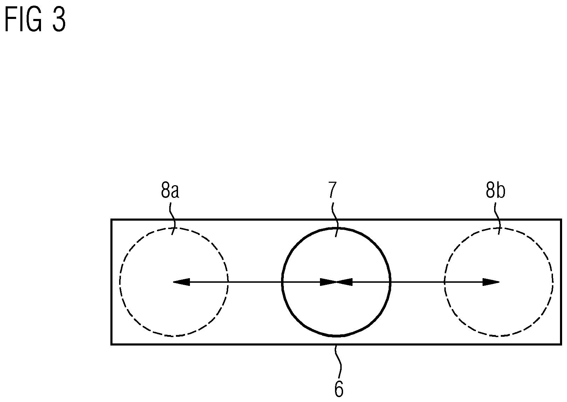

FIG 3 shows schematically the motion of a tumor 7 during the respiratory cycle. The tumor 7 moves in a range of motion 6, wherein the extremal positions 8a, 8b are reached at the maximum inhale and the maximum exhale respiratory phase.

In several embodiments, the requirements on geometric accuracy are most important for these two time intervals, since their impact on contouring accuracy is the highest. During the scan, a rule-based phase prediction algorithm, for example such as the one described in DE 10 2016 202 605 A1, may be used for real-time prediction of the patient's respiratory phase. A binary signal S may be derived as a motion state signal from the respiratory phase information, where the signal is S=1 if the patient is in a respiratory phase corresponding to one of the M selected time intervals, and S=0 otherwise. This allows to modulate the tube current according to the binary signal. For example, the full tube current may be used while S=1 and a reduced tube current may be applied while S=0. The reduction factor F of the tube current may for example be F=5 or more, such that dose is greatly reduced during the tube current modulation.

In some embodiments, it may be ensured that after a transition from S=0 to S=1, the signal keeps a value of S=1 for at least the time needed to reconstruct a single 3D volume, for example 125 ms for a rotation time of 250 ms and a reconstruction angle of 180°, even if the phase information would not be any more compatible with the targeted reconstruction time point. The CT scanner keeps track of the tube current modulation over time and assigns each set of measured raw data the corresponding value of S.

In particular, for generating the 4D-volume, the N time intervals may be selected based on the recorded breathing trace during the scan, as well as the corresponding value of S. A time point selection algorithm may for example enforce that each of the M selected time intervals is within a region with S=1. Since this restriction may reduce temporal accuracy of the data, one may also choose an even longer minimum high-dose time range, during which S is forced to the value of 1, which then allows more flexibility in the time point selection and better temporal accuracy. The remaining N - M time points may be reconstructed without restrictions on the value of S and may be reconstructed based on full-dose or dose-reduced data, or a combination thereof.

After 3D-reconstruction, for example all imaging datasets 5a, 5b may be input to a denoising algorithm. However, the denoising algorithm may be designed such, that the M imaging datasets of the second group 5b are not altered by the denoising. However, they may serve as auxiliary input for denoising the other N - M imaging datasets of the first group 5a. This way, it may be ensured that, on the one hand, the high-dose imaging datasets of the second group 5b remain unaltered since they do not need to be denoised. At the same time, the full dose of those scans can be utilized to denoise the low-dose imaging datasets of the first group 5a, for which the requirements on geometric accuracy may be relaxed.

In some embodiments, F may be chosen equal to the square of the factor of typically achievable noise reduction, for example in terms of standard deviation.

In an alternative embodiment, K > N time intervals are reconstructed with the N required time intervals and additional K - N time points in between the original N time intervals. The K- N additional time intervals can then be utilized for denoising, since they will typically have an independent noise realization. They are not denoised themselves and can be discarded after the denoising step.

In yet another alternative embodiment, an average CT is reconstructed from all acquired data without time intervals selection. In particular, all acquired raw data may contribute to the average CT and consequently movements are smeared out. The average CT can then be utilized for denoising, since it will typically have an approximately independent noise realization and a very low noise level. It is not denoised itself and can be discarded after the denoising step.

In some embodiments, the imaging apparatus 3 is a CBCT-apparatus. The described method may also be used for 4D-CBCT, since CBCT is a special form of CT and constrained equally by dose and noise.

In some embodiments, the imaging apparatus 3 is an MRI system. The described method may also be used for 4DMRI, where imaging dose plays not an important role, but the noise-affecting imaging parameter is a parameter concerning the acquisition or reconstruction time. Lower acquisition times result in lower spatial resolution. Similarly, sub-sampling or deep learning methods to increase reconstruction may have similar adverse effects. This lower spatial resolution can be restored, for example, by deep learning approaches. This allows to acquire M phases with full resolution and acquisition time, while N - M phases are acquired faster and with image resolution restored by a deep learning approach.

In several embodiments, the invention may considerably reduce the imaging dose and/or increase SNR while maintaining geometric accuracy of the most critical time points M. This overcomes restrictions of both 4DCT denoising (geometric accuracy) as well as dose-modulated scans (limited data availability) by combining both and producing a complete 4D-volume with full geometric accuracy where this is needed and reduced noise and/or dose.

Independent of the grammatical term usage, individuals with male, female or other gender identities are included within the term.

## Claims

1. Computer-implemented method for generating a reconstructed four-dimensional, 4D-, volume in time-resolved medical imaging, wherein
- a plurality of temporally ordered imaging datasets (5a, 5b) representing an imaged object and corresponding to a motion of the object is received;
- each imaging dataset of the plurality of imaging datasets (5a, 5b) is assigned to a first group (5a) or to a second group (5b) depending on a noise-affecting imaging parameter used for generating the respective imaging dataset;
- each imaging dataset of the first group (5a) is denoised using a denoising algorithm; and
- a reconstructed 4D-volume of the object is generated based on the denoised imaging datasets of the first group (5a) and based on the imaging datasets of the second group (5b).

2. Computer-implemented method according to claim 1, wherein the denoising algorithm is applied to input data, which contains at least one of the imaging datasets of the first group (5a) and at least one of the imaging datasets of the second group (5b).

3. Computer-implemented method according to claim 2, wherein the denoising algorithm comprises a trained machine learning model for denoising in medical imaging.

4. Computer-implemented method according to one of the preceding claims, wherein, for each imaging dataset of the first group (5a)
- all remaining imaging datasets of the plurality of imaging datasets (5a, 5b) are registered to the respective imaging dataset of the first group (5a); and
- denoising the respective imaging dataset of the first group (5a) comprises computing a weighted average of the respective imaging dataset of the first group (5a) and the registered imaging datasets.

5. Computer-implemented method according to one of the preceding claims, wherein
- the plurality imaging datasets correspond to X-ray images or to computed tomography, CT-, datasets and the noise-affecting imaging parameter concerns an X-ray dose used for generating the respective imaging dataset; or
- the plurality imaging datasets correspond to magnetic resonance imaging, MRI-, datasets and the noise-affecting imaging parameter concerns an acquisition time used for generating the respective imaging dataset.

6. Method for time-resolved medical imaging, wherein
- a plurality of temporally ordered imaging datasets (5a, 5b) representing an imaged object is generated during the motion of the object; and
- a computer-implemented method according to one of the preceding claims is carried out.

7. Method according to claim 6, wherein the object is a patient, and the motion corresponds to a respiratory motion of the patient.

8. Method according to one of claims 6 or 7, wherein
- a motion state signal is generated indicating a current motion state of the motion and the noise affecting imaging parameter is modulated depending on the motion state signal during the generation of the plurality of imaging datasets (5a, 5b); and
- the imaging datasets of the plurality of imaging datasets (5a, 5b) are assigned to the first group (5a) or to the second group (5b) depending on a respective value of the motion state signal.

9. Method according to claim 7 and claim 8, wherein
- the motion state signal is generated to assume either a first value or a second value;
- the motion state signal is generated to assume the second value during a maximum inhale phase of the respiratory motion and/or during a maximum exhale phase of the respiratory motion and/or during a phase halfway between the maximum exhale phase and the maximum inhale phase; and
- if the respective value is equal to the second value, then the respective imaging dataset is assigned to the second group (5b).

10. Method according to claim 9, wherein, if the respective value is equal to the first value, then the respective imaging dataset is assigned to the first group (5a).

11. Method according to one of claims 8 to 10, wherein the motion state signal is generated such that a resulting number of imaging datasets of the second group (5b) is less than a resulting number of imaging datasets of the first group (5a).

12. Method according to one of claims 6 to 11, wherein the plurality imaging datasets is generated as X-ray images or as CT-datasets by means of an X-ray imaging system and the imaging parameter is a tube current of an X-ray tube of the X-ray imaging system.

13. Data processing system (4) adapted to carry out a computer-implemented method according to one of claims 1 to 5.

14. System (1) for time-resolved medical imaging comprising a data processing system (4) according to claim 13 and an imaging apparatus (2), which is configured to generate the plurality of temporally ordered imaging datasets.

15. Computer program product comprising
- instructions, which, when executed by a data processing system (4), cause the data processing system (4) to carry out a computer-implemented method according to one of claims 1 to 5; and/or
- further instructions, which, when executed by a system (1) according to claim 14, cause the system (1) to carry out a method according to one of claims 6 to 12.
